(19) Europäisches Patentamt / European Patent Office / Office européen des brevets

(11) EP 1 839 654 B1

(12) **EUROPÄISCHE PATENTSCHRIFT**

(45) Veröffentlichungstag und Bekanntmachung des Hinweises auf die Patenterteilung:
**28.04.2010 Patentblatt 2010/17**

(51) Int Cl.:
***A61K 31/135*** *(2006.01)* ***A61K 31/415*** *(2006.01)*
***A61K 31/341*** *(2006.01)* ***A61P 29/00*** *(2006.01)*

(21) Anmeldenummer: **07014417.5**

(22) Anmeldetag: **18.11.2003**

(54) **Verwendung von (1R, 2R)-3-(3-Dimenthylamino-1-ethyl-2-methyl-propyl)-phenol zur Behandlung von Entzündungsschmerz**

Use of (1R, 2R)-3-(3-dimenthyl amino-1-ethyl-2-methyl-propyl) phenol for treating inflammatory pain

Utilisation de (1R, 2R)-3-(3-dimenthylamino-1-éthyl-2-méthyl-propyl)-phénol destiné au traitement de la douleur inflammatoire

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **22.11.2002 DE 10254785**
**06.06.2003 DE 10326103**

(43) Veröffentlichungstag der Anmeldung:
**03.10.2007 Patentblatt 2007/40**

(62) Dokumentnummer(n) der früheren Anmeldung(en) nach Art. 76 EPÜ:
**03775368.8 / 1 562 567**

(73) Patentinhaber: **Grünenthal GmbH**
**52099 Aachen (DE)**

(72) Erfinder:
- **Schiene, Klaus, Dr.**
  **40227 Düsseldorf (DE)**
- **Haase, Günter**
  **52159 Roetgen (DE)**
- **Kögel, Babette-Yvonne, Dr.**
  **52379 Langerwehe-hamich (DE)**
- **Friiderichs, Elmar, Dr.**
  **52223 Stolberg (DE)**
- **Jahnel, Ulrich, Dr.**
  **42855 Remscheid (DE)**

(56) Entgegenhaltungen:
**EP-A1- 0 693 475**
**WO-A-02/067651**
**WO-A-03/035053**
**WO-A-03/103650**
**WO-A-03/105808**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 839 654 B1

**Beschreibung**

Die Behandlung chronischer und nicht chronischer Schmerzzustände hat in der Medizin eine große Bedeutung. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für die Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Auch wenn die für die Schmerzbehandlung üblicherweise eingesetzten Analgetika - wie z. B. Opioiden, NA- und 5HT-Reuptake-Inhibitoren, NSAIDS wie auch COX-Inhibitoren - analgetisch wirksam sind, so treten doch - insbesondere bei den wirkstärkeren Opioiden - auch manchmal Nebenwirkungen auf.

Aufgabe der vorliegenden Erfindung war es daher, Stoffe aufzufinden, die zur Behandlung von Schmerz geeignet sind und bei den wirksamen Dosen bevorzugt gleichzeitig geringere Nebenwirkungen zeigen als aus dem Stand der Technik bekannt.

Die Verwendung von (1R,2R)-3-(3-Dimethylamino-1-ethyl-2-methyl-propyl)-phenol zur Schmerzbekämpfung ist aus WO 02/067651 und EP 0693457 bekannt. Keines der Dokumente nennt die Verwendung zur Herstellung eines Medikamens zu Behandlung von Entzündungsschmerz.

Überraschenderweise wurde nun gefunden, dass 3-(3-Dimethylamino-1-ethyl-2-methyl-propyl)- phenol zur Verwendung bei der Behandlung von Entzündungsschmerz geeignet ist.

Dementsprechend ist Erfindungsgegenstand ein Wirkstoff ausgewählt aus einer analgetischen **Verbindung A,** mit **Verbindung A** ausgewählt aus:

**Gruppe a)** enthaltend:

1-Phenyl-3-dimethylamino-propanverbindungen gemäß allgemeiner **Formel I**

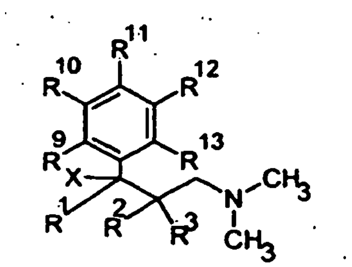

**I**

worin

X ausgewählt ist aus OH, F, Cl, H oder OC(O)$R^7$ mit $R^7$ ausgewählt aus $C_{1-3}$-Alkyl, verzweigt oder unverzweigt, gesättigt oder ungesättigt, unsubstituiert oder ein- oder mehrfach substituiert,

$R^1$ ausgewählt ist aus $c_{1-4}$-Alkyl, verzweigt oder unverzweigt, gesättigt oder ungesättigt, unsubstituiert oder ein- oder mehrfach substituiert,

$R^2$ und $R^3$ jeweils unabhängig voneinander ausgewählt sind aus H oder $C_{1-4}$-Alkyl, verzweigt oder unverzweigt, gesättigt oder ungesättigt, unsubstituiert oder ein- oder mehrfach substituiert,

oder

$R^2$ und $R^3$ zusammen einen gesättigten $C_{4-7}$-Cycloalkylrest bilden, unsubstituiert oder ein- oder mehrfach substituiert,

$R^9$ bis $R^{13}$ jeweils unabhängig voneinander ausgewählt sind aus H, F, Cl, Br, I, $CH_2F$, $CHF_2$, $CF_3$, OH, SH, $OR^{14}$, $OCF_3$, $SR^{14}$, $NR^{17}R^{18}$, $SOCH_3$, $SOCF_3$; $S0_2CH_3$, $S0_2CF_3$, CN, $COOR^{14}$, $N0_2$, $CONR^{17}R^{18}$; $C_{1-6}$-Alkyl, verzweigt oder unverzweigt, gesättigt oder ungesättigt, unsubstituiert oder ein- oder mehrfach substituiert; Phenyl, unsubstituiert oder ein- oder mehrfach substituiert;

mit $R^{14}$ ausgewählt aus $C_{1-6}$-Alkyl; Pyridyl, Thienyl, Thiazolyl, Phenyl, Benzyl oder Phenethyl, jeweils unsubstituiert oder ein- oder mehrfach substituiert;

PO(O-$C_{1-4}$-Alkyl)$_2$, CO(O$C_{1-5}$-Alkyl), CONH-$C_6H_4$-($C_{1-3}$-Alkyl), CO($C_{1-5}$-Alkyl), CO-CH$R^{17}$-NH$R^{18}$, CO-$C_6H_4$-$R^{15}$, mit $R^{15}$ ortho-OCO$C_{1-3}$-Alkyl oder meta- oder para-$CH_2N(R^{16})_2$ mit $R^{16}$ $C_{1-4}$-Alkyl oder 4-Morpholino, wobei in den Resten $R^{14}$, $R^{15}$ und $R^{16}$ die Alkylgruppen verzweigt oder unverzweigt, gesättigt oder ungesättigt,

unsubstituiert oder ein- oder mehrfach substituiert sein können;
mit $R^{17}$ und R18 jeweils unabhängig voneinander ausgewählt aus H; $C_{1-6}$-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder

oder

$R^9$ und $R^{10}$ oder $R^{10}$ und $R^{11}$ zusammen einen $OCH_2O$-, $OCH_2CH_2O$-, OCH=CH-, CH=CHO-, CH=C(CH3)O-, OC (CH3)=CH-, $(CH_2)_4$- oder OCH=CHO- Ring bilden, wobei Verbindung A im Sinne dieser Anmeldung auf 3-(3- Dimethylamino- 1- ethyl- 2- methyl- propyl)- phenol, bevorzugt (1 R, 2R)- 3-(3- Dimethylamino- 1- ethyl- 2- methyl- propyl)- phenol beschränkt ist,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10- Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder-C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3- C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH, $OCH_3$, $SCH_3$, $N(CH_3)_2$, $NHCH_3$ oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von -CH (OH)-CH=CH-$CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, $-CH_2-CH_2-$, $-CH_2-CH_2-CH_2-$ und $-CH_2-CH_2-CH_2-CH_2-$ zu verstehen, unter $(CH_2)_{4-5}$ ist $-CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls

oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{23}$ und $R^{24}$ bedeuten zusammen $CH_2CH_2OCH_2CH_2$, $CH_2CH_2NR^{25}CH_2CH_2$ oder $(CH_2)_{3-6}$, und der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/ oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure. Besonders bevorzugt ist das Hydrochlorid-Salz und das Citrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro 1$\lambda^6$ -benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium-oder Calzium-Salze.

Verbindungen der **Gruppe a)** und deren Herstellung sind aus der DE 44 26 245 A1 bekannt.

In einer bevorzugten Ausführungsform gilt für **Verbindung A** ausgewählt aus Gruppe a), die ausgewählt aus Verbindungen gemäß **Formel I** ist, für die gilt:

X ausgewählt ist aus
OH, F, Cl, $OC(O)CH_3$ oder H, vorzugsweise OH, F, $OC(O)CH_3$ oder H,

**und/oder**

$R^1$ ausgewählt ist aus
$C_{1-4}$-Alkyl, gesättigt und unsubstituiert, verzweigt oder unverzweigt; vorzugsweise $CH_3$, $C_2H_5$, $C_4H_9$ oder t-Butyl, insbesondere $CH_3$ oder $C_2H_5$,

**und/oder**

$R^2$ und $R^3$ unabhängig voneinander ausgewählt sind aus
H, $C_{1-4}$-Alkyl, gesättigt und unsubstituiert, verzweigt oder unverzweigt; vorzugsweise H, $CH_3$, $C_2H_5$, i-Propyl oder t-Butyl, insbesondere H oder $CH_3$, vorzugsweise $R^3$ = H,
oder
$R^2$ und $R^3$ zusammen einen $C_{5-6}$-Cycloalkylrest bilden, gesättigt oder ungesättigt, unsubstituiert oder ein- oder mehrfach substituiert, vorzugsweise gesättigt und unsubstituiert, insbesondere Cyclohexyl.

**und/oder**

$R^9$ bis $R^{13}$, wobei 3 oder 4 der Reste $R^9$ bis $R^{13}$ H entsprechen müssen, unabhängig voneinander ausgewählt sind aus H, Cl, F, OH, $CF_2H$, $CF_3$ oder $C_{1-4}$-Alkyl, gesättigt und unsubstituiert, verzweigt oder unverzweigt; $OR^{14}$ oder $SR^{14}$, mit $R^{14}$ ausgewählt aus $C_{1-3}$-Alkyl, gesättigt und unsubstituiert, verzweigt oder unverzweigt;
vorzugsweise H, Cl, F, OH, $CF_2H$, $CF_3$, $OCH_3$ oder $SCH_3$

oder $R^{12}$ und $R^{11}$ einen 3,4-OCH=CH-Ring bilden
insbesondere

wenn $R^9$, $R^{11}$ und $R^{13}$ H entsprechen, einer von $R^{10}$ oder $R^{12}$ auch H entspricht, während der andere ausgewählt ist aus:

Cl, F, OH, $CF_2H$, $CF_3$, $OR^{14}$ oder $SR^{14}$, vorzugsweise OH, $CF_2H$, $OCH_3$ oder $SCH_3$

oder,

wenn $R^9$ und $R^{13}$ H entsprechen und $R^{11}$ OH, $OCH_3$, Cl oder F, vorzugsweise Cl, entspricht, einer von $R^{10}$ oder $R^{12}$ auch H entspricht, während der andere OH, $OCH_3$, Cl oder F, vorzugsweise Cl, entspricht,

oder,

wenn $R^9$, $R^{10}$, $R^{12}$ und $R^{13}$ H entsprechen, $R^{11}$ ausgewählt ist aus $CF_3$, $CF_2H$, Cl oder F, vorzugsweise F,

oder,

wenn $R^{10}$, $R^{11}$ und $R^{12}$ H entsprechen, einer von $R^9$ oder $R^{13}$ auch H entspricht, während der andere ausgewählt ist aus OH, $OC_2H_5$ oder $OC_3H_7$.

Dabei gilt für Verbindungen der **Gruppe a)**, daß Verbindungen der **Formel I** mit $R^3$ = H in Form der Diastereomeren mit der relativen Konfiguration Ia

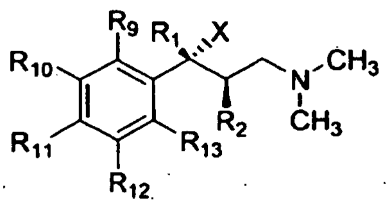

**Ia**

vorliegen, insbesondere in Mischungen mit höherem Anteil dieses Diastereomeren im Vergleich zum anderen Diastereomeren oder als reines Diastereomer verwendet werden.

Dabei gilt für Verbindungen der **Gruppe a)**, daß **Verbindung A** ausgewählt aus folgender Gruppe verwendet wird:

(2RS,3RS)-1-Dimethylamino-3-(3-methoxy-phenyl)-2-methyl-pentan-3-ol,
(+)-(2R,3R)-1-Dimethylamino-3-(3-methoxy-phenyl)-2-methyl-pentan-3-ol,
(2RS,3RS)-3-(3,4-Dichlorophenyl)-1-dimethylamino-2-methyl-pentan-3-ol,
(2RS,3RS)-3-(3-Difluoromethyl-phenyl)-1-dimethylamino-2-methyl-pentan-3-ol,
(2RS,3RS)-1-Dimethylamino-2-methyl-3-(3-methylsulfanyl-phenyl)-pentan-3-ol,
(3RS)-1-Dimethylamino-3-(3-methoxy-phenyl)-4,4-dimethyl-pentan-3-ol,
(2RS,3RS)-3-(3-Dimethylamino-1-ethyl-1-hydroxy-2-methylpropyl)-phenol,
(1 RS,2RS)-3-(3-Dimethylamino-1-hydroxy-1,2-dimethyl-propyl)-phenol,
(+)-(1 R,2R)-3-(3-Dimethylamino-1-hydroxy-1,2-dimethylpropyl)-phenol,
(+)-(1 R,2R)-3-(3-Dimethylamino-1-hydroxy-1,2-dimethyl-propyl)-phenol,
(-)-(1 R,2R)-3-(3-Dimethylamino-1-ethyl-2-methyl-propyl)-phe-nol,
(+)-(1 R,2R)-Essigsäure-3-dimethylamino-1-ethyl-1-(3-methoxy-phenyl)-2-methyl-propylester,
(1 RS)-1-(1-Dimethylaminomethyl-cyclohexyl)-1-(3-methoxyphenyl)-propan-1-ol,
(2RS, 3RS)-3-(4-Chlorophenyl)-1-dimethylamino-2-methyl-pentan-3-ol,
(+)-(2R,3R)-3-(3-Dimethylamino-1-ethyl-1-hydroxy-2-methyl-propyl)-phenol,
(2RS,3RS)-4-Dimethylamino-2-(3-methoxy-phenyl)-3-methyl-butan-2-ol und
(+)-(2R,3R)-4-Dimethylamino-2-(3-methoxy-phenyl)-3-methyl-butan-2-ol,

vorzugsweise als Hydrochlorid.

Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutan Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäßen Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0,05 bis 90,0 % der Wirkstoffkombination enthalten, insbesondere niedrige wirksame Dosierungen, um Neben- oder analgetische Wirkungen zu vermeiden. Üblicherweise werden 0,1 bis 5000 mg/kg, insbesondere 1 bis 500 mg/kg, vorzugsweise 2 bis 250 mg/kg Körpergewicht der Kombination appliziert. Ebenso bevorzugt und üblich ist aber auch die Applikation von 0,01 - 5 mg/kg, vorzugsweise 0,03 bis 2 mg/kg, insbesondere 0,05 bis 1 mg/kg Körpergewicht.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellutose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaürylsulfat, genießbare Öle, Sesamöl, Kokusnußöl, Erdnußöl, Sojaböhnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington’s Pharmaceutical Sciences", Hrsg. A.R. Gennaro, 17. Ed., Mack

Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke; Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die Wirkstoff in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Ein weiterer Gegenstand der Erfindung ist die Verwendung eines erfindungsgemäßen Wirkstoffes A zur Behandlung von Entzündungsschmerz.

Weiter betrifft die Erfindung auch ein Verfahren zur Behandlung von Entzündungsschmerz bei dem die erfindungsgemäße Wirkstoffes A verwendet wird.

Weiterer Gegenstand der Erfindung ist Verbindung A zur Verwendung bei der Behandlung von Entzündungsschmerz. Im Sinne dieser Anmeldung wird Verbindung A als 3-(3-Dimethylamino-1-ethyl-2-methyl-propyl)- phenol, bevorzugt (1 R,2R)- 3-(3-Dimethylamino-1-ethyl-2-methyl-propyl)- phenol, bevorzugt als Hydrochlorid-Salz verstanden. Weiterer Gegenstand ist, dass (1 R,2R)- 3-(3-Dimethylamino-1-ethyl-2-methyl-propyl)- phenol in Form ihrer Säure oder Base oder in Form ihrer Salze, oder in Form ihrer Solvate, insbesondere der Hydrate vorliegen kann, wobei als Salz physiologisch verträgliche Salze bevorzugt sind, wobei die Verbindung sowohl als racemisches Gemisch oder in Form ihrer Enantiomeren rein oder im Gemisch vorliegen kann und zur Behandlung von Entzündingsschmerz eingesetzt wird. Weiterer Gegenstand ist die Verwendung der erfindungsgemäßen Verbindung A zur Herstellung eines Medikaments zur Behandlung von Entzündungsschmerz.

**Beispiele:**

Die folgenden Beispiele sollen die Erfindung erläutern, ohne daß der Gegenstand der Erfindung darauf beschränkt wäre.

**Beispiel 1: Supra-additive Wirkung im Randall-Selitto-Test:**

Nachfolgend sind die auf ihre Wirksamkeit getesteten Verbindungen aufgelistet. Die Nummerierung wird in den Beispielen und den Abbildungen gebraucht:

**Verbindung A:**

| Name | Verbdg. Nr. |
|---|---|
| (1R,2R)-3-(2-Dimethylaminomethyl-cyclohexyl)-phenol;Hydrochlorid Hydrochlorid | **1** |
| (1R,2R)-3-(2-Dimethylaminomethyl-1-ethyl-2-methyl-propyl)-phenol; | **2** |
| (1S,2S)-3-(3-Dimethylamino-1-ethyl-2-methyl-propyl)- phenol; Hydrochlorid | **3** |
| (1RS,3RS, 6RS)-6-Dimethylaminomethyl-1-(3-[methoxy-phenyl)-cyclohexane-1,3-diol; Hydrochlorid | **4** |

**COX.II-Inhibitoren:**

| Name | Verbdg. Nr. |
|---|---|
| Celecoxib | **101** |
| Rofecoxib | **102** |

(fortgesetzt)

| Name | Verbdg. Nr. |
|---|---|
| Celecoxib in Form des Fertigarzneimittels CELEBREX® | **103** |
| Rofecoxib in Form des Fertigarzneimittels VIOXX® | **104** |

**Beispiel 2: Writhing-Test Maus**

Der Writhing-Test stellt eine Modifikation der Methode von Schmauss C and Yaksh TL, J Pharmacol Exp Ther 228: 1-12 (1984) und ein Modell für den Akutschmerz dar.

Die Tiere erhalten als Schmerzstimulus 9% Essigsäure intraperitoneal appliziert. Diese wird 30min (Testsubstanz p.o.) oder 10min (Testsubstanz i.v.) nach der Gabe der Testsubstanz appliziert. Registriert werden die Schmerzinduzierten Writhingreaktionen 10-35 min nach der Essigsäure-Gabe. Da die Ratte selten die klassische Writhingreaktion der Maus zeigt wohl aber andere sehr deutliche Schmerzäußerungen, erfolgt die Bewertung durch eine Score-Einteilung. Schmerzäußerungen wie z.B. Schiefneigung des Körpers beim stehenden Tier, häufiges Putzen des Bauches oder Katzenbuckel ohne Abstrecken der Hintergliedmaßen gehen so in die Bewertung ein.

Verglichen werden die substanzbehandelten Tiere mit einer Kontrollgruppe, die phys. Kochsalzlösung erhielt. Die Gruppengröße beträgt 10 Tiere (n=10).

Das Ergebnis der Wirkstoffkombination von Verbindung Nr. 2 mit den COX II -Inhibitoren 101, 102, 103 und 104 ist Tabelle 1 zu entnehmen.

**Beispiel 3: Formalin-Test**

Der Formalin Test (Dubuisson, D. and Dennis, S.G., 1977, Pain, 4, 161 - 174) stellt ein Modell für den akuten sowie den chronischen Schmerz dar. In den hier vorgestellten Untersuchungen wurde die chronische Schmerzkomponente ausgewertet.

Durch eine einmalige Formalin-Injektion in die dorsale Seite einer Hinterpfote wird bei freibeweglichen Versuchstieren eine biphasische nociceptive Reaktion induziert, die durch Beobachtung von drei deutlich voneinander unterscheidbaren Verhaltensmustern erfaßt wird.

Formalin wird mit dem Volumen von 20 µl und einer Konzentration von 1 % subkutan in die dorsale Seite der rechten Hinterpfote jedes Tieres appliziert. Die spezifischen Verhaltensänderungen, wie Anheben und Schütteln der Pfote, Gewichtsverlagerungen des Tieres sowie Beiss- und Leckreaktionen werden bis 60 min nach Formalinapplikation kontinuierlich beobachtet und registriert. Die Verhaltensänderungen werden unterschiedlich gewichtet (Score 0-3) und eine Pain-Rate (PR) errechnet mit folgender Formel:

$$PR = [(T_0 \times 0) + (T_1 \times 1) + (T_2 \times 2) + (T_3 \times 3) / 180.$$

Dabei entsprechen $T_0$, $T_1$, $T_2$, $T_3$ jeweils der Zeit in Sekunden, in der das Tier die Verhaltensweisen 0, 1, 2, oder 3 zeigte. Die Gruppengröße beträgt 10 Tiere (n=10).

Das Ergebnis der Wirkstoffkombination von Verbindung Nr. 2 mit den COX II -Inhibitoren 101, 102, 103 und 104 ist Tabelle 1 zu entnehmen.

**Beispiel 4: Randell-Selitto-Test**

Der Randall-Selitto-Test nach Randall und Selitto (Arch. Int. Pharmacodyn., 1957, 111:409-419) stellt ein Modell für den Entzündungsschmerz dar. Gegenstand der Anmeldung ist daher die Verordnung des Verbindung A zw Behandlung von Entzündungsschmerz.

Durch Injektion von 0,1 ml 20 %ige Bäckerhefe-Suspension ventral in eine Hinterpfote wird ein Ödem induziert, an dem 4h später durch kontinuierlich ansteigendem Druck mit einem Stempel (2 mm Spitzendurchmesser) ein Schmerz erzeugt wird. Der zu bestimmende Messwert und gleichzeitig auch der Endpunkt des Schmerztestes ist der Druck bei dem die Vokalisationsreaktion des Tieres auftritt. Berechnet wird der prozentuale maximale mögliche Effekt ("maximal possible effect", %MPE). Dabei beträgt der maximale Druck des Stempels 250g. Die Gruppengröße beträgt n=10.

Die Ergebnisse der Versuche sind den Abbildungen 1 bis 4 sowie den nachfolgenden Tabellen 1 bis 4 zu entnehmen.

**Auswertung:**

Die Berechnung des theoretischen additiven Wertes erfolgt durch die Addition der Mittelwerte aus den Einzeluntersuchungen von Testsubstanz und COX-II Inhibitor. Die entsprechende Standardabweichung des theoretischen additiven Mittelwertes wurde aus der Addition der Varianzen berechnet.

Die ausgewählten Analgetika (Verbindung A) zeigten in allen oben aufgeführten Tiermodellen eine mittelstarke bis starke Hemmung der Nociception bzw. der Hyperalgesie.

Die simultane Applikation von einem selektiven COX-II-Inhibitor (Rofecoxib oder Celecoxib) mit der Verbindung 1 führte in den aufgelisteten Versuchen, also sowohl im Akut-, im chronischem- und im EntzündungsSchmerz (Tab. 1) zu einer Wirkverstärkung im Vergleich zur theoretischen errechneten rein additiven Wirkung. Hervorzuheben ist ein deutlicher wirkungssteigender supraadditiver Effekt bei zeitversetzter Gabe von COX-II-Inhibitor und den ausgewählten Analgetika (Verbindung A) Verbindungen Nr. 1, 4, 2 und 3 (Randall-Selitto-Test). Bei diesen Versuchen wurde entweder Rofecoxib oder Celecoxib eine Stunde vor der Applikation des ausgewählten Analgetikums (Verbindung A) gegeben. In einigen Untersuchungen wurde Rofecoxib als Fertigpräparat Vioxx und Celecoxib als Celebrex appliziert.

Die Ergebnisse ausgewählter Untersuchungen hier beispielsweise mit der Verbindung Nr. 1 sind in der nachfolgenden Tabelle (Tabelle 1) zusammengefasst. Fixed ratio combination: equieffektive Dosierung der beiden Substanzen, errechnet aus dem Verhältnis der jeweiligen ED50-Werte. Fixed dose combination: feste Dosierungen der jeweiligen Substanzen.

Tabelle 1:

| Test (Tiermodell) | Verbindgn. Nr. / Kombinat. (Vbdg. A + COX II) | Art der Applikation | Ratio der Verbindung A (1) zum COX-II-Inhibitor (2) 1: | 2: | Dosierung der Verbindung A (1) und des COX-II-Inhibitors (2) [mg/kg] 1: | 2: | % Wirkung / Ed50 [mg/kg] der Verbindung A | % Wirkung / Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| **Fixed ratio combination** | | | | | | | | | | |
| Writhing (Ratte) | 2 + 102 | p.o. | 1 | 2 | - | - | $ED_{50}$: 33,0 | $ED_{50}$: 60,1 | $ED_{50}$: 47,0 | $ED_{50}$: 23,3 |
| Formalintes (Maus) | 2+101 | p.o. | 47,3 | 52,7 | - | - | $ED_{50}$: 34,6 | $ED_{50}$: 38,5 | $ED_{50}$: 36,5 | $ED_{50}$: 22,8 |
| Formalintes (Ratte) | 2 + 101 | p.o. | 10,5 | 89,5 | - | - | $ED_{50}$:18,0 | $ED_{50}$: 154 | $ED_{50}$: 86,0 | $ED_{50}$: 60,6 |
| | | | | | | | | | | |
| **Fixed dose combination:** | | | | | | | | | | |
| Randall-Selitto (Ratte) | 2 + 104 | p.o. | - | - | 31 | 215 | 13,3 | 11,2 | 24,5 | 29,7 |
| Randall--Selitto (Ratte) | 2 + 101 | p.o. | - | - | 31 | 215 | 13,3 | 9,8 | 23,1 | 29,6 |
| **Fixed dose combination (zeitversetzte Gabe; COX-Inhibitor 1h vor Verbindung A):** | | | | | | | | | | |
| Randall-Selitto (Ratte) | 2 + 104 | p.o. | - | - | 31 | 215 | 20,0 | 13,0 | 33,0 | 45,7 |

(fortgesetzt)

| **Fixed dose combination (zeitversetzte Gabe; COX-Inhibitor 1h vor Verbindung A):** | | | | | | | | | | |
|---|---|---|---|---|---|---|---|---|---|---|
| Randall-Selitto (Ratte) | 2+ 104 | p.o. | - | - | 46 | 215 | 32,6 | 13,0 | 45,6 | 59,8 |
| Randall-Selitto (Ratte) | 2 + 103 | p.o. | - | - | 31 | 215 | 20,0 | 17,3 | 37,3 | 60,9 |
| Randall-Selitto (Ratte) | 2 + 103 | p.o. | - | - | 46 | 215 | 32,6 | 17,3 | 49,9 | 86,1 |

Tabelle 2 zeigt die Versuche gemäß Beispiel 4 mit Verbindung Nr 4.

**Tabelle 2:**

| Test (Tiermodell) | Verbindgn. Nr. / Kombinat (Vbdg. A + COX II) | Art der Applikation | Ratio der Verbindung A (1) zum COX-II-Inhibitor (2) 1: | 2: | Dosierung der Verbindung A (1) und des COX-II-Inhibitors (2) [mg/kg] 1: | 2: | % Wirkung / Ed50 [mg/kg] der Verbindung A | % Wirkung / Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| **Fixed dose combination (zeitversetzte Gabe; COX-Inhibitor 1h vor Verbindung A):** | | | | | | | | | | |
| RandallSelitto (Ratte) | 4+103 | p.o. | - | - | 68,1 | 215 | 85,4 15-75 min* | 16,7 15-75 min | 102,1 15-75 min | 109,3 15-75 min |
| Randall-Selitto (Ratte) | 4+ 103 | p.o. | - | - | 68,1 | 215 | 45,2 75-90 min* | 8,0 76-90 min | 53,2 75 - 90 min | 68,5 75 - 90 min |
| * = Ausgewerteter Zeitraum nach Gabe von Verbindung Nr. 4 (s. Abb. 2) | | | | | | | | | | |

Tabelle 3 zeigt die Versuche gemäß Beispiel 4 mit Verbindung Nr 1.

**Tabelle 3:**

| Test (Tiermodell) | Verbindgn. Nr. / Kombinat. (Vbdg. A + COX II) | Art der Applikation | Ratio der Verbindung A (1) ; zum COX-II-Inhibitor (2) | | Dosierung der Verbindung A (1) und des COX-II-Inhibitors (2) [mg/kg] | | % Wirkung /Ed50 [mg/kg] der Verbindung A | % Wirkung /Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| | | | 1: | 2: | 1: | 2: | | | | |
| **Fixed dose combination (zeitversetzte Gabe; COX-Inhibitor 1h vor Verbindung A):** | | | | | | | | | | |
| Randall-Selitto (Ratte) | 1+103 | p.o. | - | - | 46,4 | 215 | 26,2 | 8,9 | 35,1 | 84,1 |

**Tabelle 4:**

| Test (Tiermodell) | Verbindgn. Nr. / Kombinat. (Vbdg. A + COX II) | Art der Applikation | Ratio der Verbindung A (1) zum COX-II-Inhibitor (2) | | Dosierung der Verbindung A (1) und des COX-II-Inhibitors (2) [mg/kg] | | % Wirkung /Ed50 [mg/kg] der Verbindung A | % Wirkung / Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| | | | 1: | 2: | 1: | 2: | | | | |
| **Fixed dose combination (zeitversetzte Gabe; COX-Inhibitor 1h vor Verbindung A):** | | | | | | | | | | |
| Randall-Selitto (Ratte) | 3+103 | p.o. | - | - | 21,5 | 215 | 22,8 | 8,9 | 31,7 | 96,4 |

**Beispiel 6: Parenterale Applikationsform**

10 g (1R,2R)-3-(2-Dimethylaminomethyl-cyclohexyl)-phenol;Hydrochlorid und 20 g Celecoxib werden in 1 l Wasser für Injektionszwecke bei Raumtemperatur gelöst und anschließend durch Zugabe von NaCl auf isotone Bedingungen eingestellt.

## Patentansprüche

1. (1R,2R)-3-(3-Dimethylamino-1-ethyl-2-methylpropyl)- phenol zur Verwendung bei der Behandlung von Entzündungsschmerz.

2. Verwendung von (1R,2R)-3-(3-Dimethylamino-1-ethyl-2-methylpropyl)- phenol zur Herstellung eines Medikaments zur Behandlung von Entzündungsschmerz.

3. Verwendung gemäß Anspruch 2 wobei (1R,2R)-3-(-3-Dimethylamino-1-ethyl-2-methyl-propyl)- phenol in Form ihrer Säure oder Base oder in Form ihrer Salze, insbesondere der physiologisch verträglichen Salze, oder in Form ihrer Solvate insbesondere der Hydrate vorliegen kann, wobei die Verbindung sowohl als racemisches Gemisch oder in Form Ihrer Enantiomeren rein oder im Gemisch vorliegen kann.

## Claims

1. (1R,2R)-3-(3-dimethylamino-1-ethyl-2-methyl-propyl)-phenol for use in the treatment of pain caused by inflammation.

2. Use of (1R,2R)-3-(3-dimethylamino-1-ethyl-2-methyl-propyl)-phenol for the production of a drug for the treatment of pain caused by inflammation.

3. Use according to claim 2, wherein (1R,2R)-3-(3-dimethylamino-1-ethyl-2-methylpropyl)-phenol may be present in the form of the acid or base thereof, or in the form of the salts thereof, in particular the physiologically acceptable salts, or in the form of the solvates thereof, in particular the hydrates, it being possible for the compound to be present in pure form or in a mixture, as a racemic mixture or in the form of the enantiomers thereof.

## Revendications

1. Le (1R,2R)-3-(3-diméthylamino-1-éthyl-2-méthyl-propyl)-phénol pour son utilisation dans le traitement de la douleur inflammatoire.

2. Utilisation du (1R,2R)-3-(3-diméthylamino-1-éthyl-2-méthyl-propyl)-phénol pour la préparation d'un médicament destiné au traitement de la douleur inflammatoire.

3. Utilisation suivant la revendication 2, le (1R,2R)-3-(3-diméthylamino-1-éthyl-2-méthyl-propyl)-phénol pouvant se présenter sous sa forme d'acide ou de base ou sous forme de ses sels, en particulier des sels physiologiquement acceptables, ou sous forme de ses produits de solvatation, en particulier sous forme des hydrates, le composé pouvant être présent tant comme mélange racémique que sous forme de ses énantiomères purs ou en mélange.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 02067651 A
- EP 0693457 A
- DE 4426245 A1


### In der Beschreibung aufgeführte Nicht-Patentliteratur


- Remington's Pharmaceutical Sciences. Mack Publishing Company, 1985, vol. 8
- **SCHMAUSS C ; YAKSH TL.** *J Pharmacol Exp Ther,* 1984, vol. 228, 1-12
- **DUBUISSON, D. ; DENNIS, S.G.** *Pain,* 1977, vol. 4, 161-174
- **RANDALL ; SELITTO.** *Arch. Int. Pharmacodyn.,* 1957, vol. 111, 409-419